# NEUE EUROPÄISCHE PATENTSCHRIFT

(11) **EP 1 560 926 B2**
(45) Veröffentlichungstag und Bekanntmachung des Hinweises auf die Entscheidung über den Einspruch: **21.08.2013**
(45) Hinweis auf die Patenterteilung: 06.01.2010
(21) Anmeldenummer: 03767417.3
(22) Anmeldetag: 10.11.2003
(51) Int. Cl.: C12Q 1/68, C12N 15/10

(54) **NEUARTIGE PUFFERFORMULIERUNGEN ZUR ISOLIERUNG, REINIGUNG UND RÜCKGEWINNUNG LANG- UND KURZKETTIGER NUKLEINSÄUREN**
NOVEL BUFFER FORMULATIONS FOR ISOLATING, PURIFYING AND RECOVERING LONG-CHAIN AND SHORT-CHAIN NUCLEIC ACIDS
NOUVELLES FORMULATIONS DE TAMPONS POUR ISOLER, PURIFIER ET RECUPERER DES ACIDES NUCLEIQUES A CHAINE LONGUE ET A CHAINE COURTE

(30) Priorität: 08.11.2002 DE 10252545; 14.11.2002 DE 10253351
(43) Veröffentlichungstag der Anmeldung: 10.08.2005
(73) Patentinhaber: STRATEC Molecular GmbH, 13125 Berlin (DE)
(72) Erfinder: HILLEBRAND, Timo, 15366 Hönow (DE); BENDZKO, Peter, 12623 Berlin (DE)
(74) Vertreter: Lange, Sven
(86) Internationale Anmeldenummer: PCT/DE2003/003728
(87) Internationale Veröffentlichungsnummer: WO 2004/042058

(56) Entgegenhaltungen:
- EP-A- 0 580 305
- EP-A2- 0 580 305
- WO-A-01/19980
- WO-A-02/04620
- WO-A1-01//19980
- WO-A1-03/097831
- WO-A1-95//21849
- WO-A1-99//016869
- WO-A2-02//04620
- DE-A1- 10 033 991
- DE-A1- 10 033 991
- DE-A1- 19 856 064
- DE-U1- 20 207 793
- US-A- 5 898 071
- US-A1- 2001 021 518
- LAHIRI D K ET AL: "DNA ISOLATION BY A RAPID METHOD FROM HUMAN BLOOD SAMPLES: EFFECT OF MGCL2, EDTA, STORAGE TIME, AND TEMPERATURE ON DNA YIELD AND QUALITY" BIOCHEMICAL GENETICS, PLENUM PRESS CO., LONDON, GB, Bd. 31, Nr. 7/8, August 1993 (1993-08), Seiten 321-328, XP009007516 ISSN: 0006-2928

## Beschreibung

Die Erfindung betrifft neuartige Formulierungen von Puffern zur Isolierung, Reinigung und Rückgewinnung von lang- und kurzkettigen Nukleinsäuren.
Die Anwendungsgebiete des Verfahrens sind alle mit Nukleinsäure-Isolierungen sich beschäftigenden Laboratorien, wie forensische Medizin, Lebensmitteldiagnostik, medizinische Diagnostik, Molekularbiologie, Biochemie, Gentechnik und alle anderen angrenzenden Gebiete.

Unter klassischen Bedingungen erfolgt die Isolierung von DNA aus Zellen und Geweben dadurch, dass die Ausgangsmaterialien unter stark denaturierenden und reduzierenden Bedingungen, teilweise auch unter Verwendung von proteinabbauenden Enzymen aufgeschlossen, die austretenden Nukleinsäurefraktionen über Phenol-/Chloroform-Extraktionsschritte gereinigt und die Nukleinsäuren mittels Dialyse oder Ethanolpräzipitation aus der wäßrigen Phase gewonnen werden (Sambrook, J., Fritsch, E.F. und Maniatis, T., 1989, CSH, "Molecular Cloning").

Diese "klassischen Verfahren" zur Isolierung von Nukleinsäuren aus Zellen und besonders aus Geweben sind sehr zeitaufwendig (teilweise länger als 48 h), erfordern einen erheblichen apparativen Aufwand und sind darüber hinaus auch nicht unter Feldbedingungen realisierbar. Außerdem sind solche Methoden auf Grund der verwendeten Chemikalien wie Phenol und Chloroform in einem nicht geringen Maße gesundheitsgefährdend.

Verschiedene alternative Verfahren zur Isolierung von Nukleinsäuren aus unterschiedlichen biologischen Ausgangsmaterialien ermöglichen, die aufwendige und gesundheitsschädigende Phenol-/Chloroform-Extraktion von Nukleinsäuren zu umgehen sowie eine Reduzierung der zeitlichen Aufwendungen zu erreichen.

Alle diese Verfahren basieren auf einer von Vogelstein und Gillespie (Proc. Natl. Acad. Sci. USA, 1979, 76, 615-619) entwickelten und erstmals beschriebenen Methode zur präparativen und analytischen Reinigung von DNA-Fragmenten aus Agarosegelen. Die Methode kombiniert die Auflösung der die zu isolierende DNA- Bande enthaltende Agarose in einer gesättigten Lösung eines chaotropen Salzes (NaJ) mit einer Bindung der DNA an Glaspartikel. Die an die Glaspartikel fixierte DNA wird anschließend mit einer Waschlösung (20 mM Tris HCl [pH 7,2]; 200mM NaCl; 2 mM EDTA; 50% v/v Ethanol) gewaschen und abschließend von den Trägerpartikeln abgelöst.

Diese Methode erfuhr bis heute eine Reihe von Modifikationen und wird zum gegenwärtigen Zeitpunkt für unterschiedliche Verfahren der Extraktion und Reinigung von Nukleinsäuren aus unterschiedlichen Herkünften angewendet (Marko, M.A., Chipperfield, R. und Bimboim, H.G., 1982, Anal. Biochem., 121, 382-387).

Darüber hinaus existieren heute weltweit auch eine Vielzahl von Reagenziensystemen vor allem zur Reinigung von DNA-Fragmenten aus Agarosegelen und für die Isolierung von Plasmid DNA aus bakteriellen Lysaten, aber auch für die Isolierung von längerkettigen Nukleinsäuren (genomische DNA, zelluläre Gesamt- RNS) aus Blut, Geweben oder auch Zellkulturen.

Alle diese kommerziell verfügbaren Kits basieren auf dem hinlänglich bekannten Prinzip der Bindung von Nukleinsäuren an mineralische Träger unter Anwesenheit von Lösungen unterschiedlicher chaotroper Salze und verwenden als Trägermaterialien Suspensionen feingemahlener Glaspulver (z.B. Glasmilk , BIO 101, La Jolla, CA), Diatomenerden (Fa.Sigma) oder auch Silicagele (Diagen, DE 41 39 664 A1).

Ein für eine Vielzahl unterschiedlicher Anwendungen praktikables Verfahren zur Isolierung von Nukleinsäuren ist in US 5,234,809 (Boom) dargestellt. Dort ist ein Verfahren zur Isolierung von Nukleinsäuren aus nukleinsäurehaltigen Ausgangsmaterialien durch die Inkubation des Ausgangsmaterials mit einem chaotropen Puffer und einer DNA-bindenden festen Phase beschrieben. Die chaotropen Puffer realisieren sowohl die Lyse des Ausgangsmaterials wie auch die Bindung der Nukleinsäuren an die feste Phase. Das Verfahren ist gut geeignet , um Nukleinsäuren aus kleinen Probenmengen zu isolieren und findet speziell im Bereich der Isolierung viraler Nukleinsäuren seine praktische Anwendung.

Entscheidende Nachteile des Verfahrens bestehen aber u.a. darin, dass der durch die chaotropen Puffer realisierte Aufschluss nicht für alle Materialien einsetzbar ist bzw. auch für größere Mengen an Ausgangsmaterialien nur extrem ineffizient und unter einem großen Zeitaufwand realisiert werden kann. Darüber hinaus sind mechanische Homogenisierungsverfahren notwendig, wenn z.B. DNA aus Gewebeproben isoliert werden soll. Weiterhin müssen für verschiedene Fragestellungen auch immer verschieden hohe Konzentrationen unterschiedlicher chaotroper Puffer eingesetzt werden. Das Verfahren ist damit in keiner Weise universell einsetzbar.

Das physiko-chemische Prinzip der nach dem bekannten Stand der Technik heute eingesetzten und kommerziell verfügbaren Systeme zur Isolierung von Nukleinsäuren auf der Basis der Bindung von Nukleinsäuren an die Oberflächen mineralischer Träger soll dabei in der Störung übergeordneter Strukturen des wässerigen Millieus bestehen, durch welche die Nukleinsäuren an der Oberfläche von mineralischen Materialien, insbesondere von Glas-bzw. Silicapartikeln adsorbieren. Die Störung der übergeordneten Strukturen des wässrigen Milieus erfolgt dabei immer unter Anwesenheit chaotroper Ionen und ist bei hohen Konzentrationen dieser fast quantitativ. Auf dieser beschriebenen physiko-chemischen Basis enthalten alle kommerziell verfügbaren Systeme zur Isolierung von Nukleinsäuren Pufferkompositionen mit hohen Ionenstärken chaotroper Salze, für die Bindung von Nukleinsäuren an eine Nukleinsäurenbindende feste Phase.

Spezifische Modifikationen dieser Verfahren betreffen den Einsatz von spezifischen Trägermaterialien, welche für bestimmte Fragestellungen applikative Vorteile zeigen (Invitek GmbH WO 95/34569), die jedoch die gleichen Nachteile aufweisen.

All den beschriebenen Verfahren zur Isolierung von Nukleinsäuren über die Bindung der Nukleinsäuren an mineralische feste Phase unter Verwendung chaotroper Salzlösungen ist gemeinsam, dass für die Bindung der Nukleinsäuren an die verwendeten Trägermaterialien hohe Konzentrationen eingesetzt werden müssen. Dabei sind gerade chaotrope Salze (z.B. Guanidinisothiocyanat, Guandinhydrochlorid, Natriumperchlorat oder Natriumjodid) hochtoxisch wirksame Substanzen. Die Verwendung findenden Puffersysteme mit sehr hohen Ionenstärken bewirken oftmals ein Verschleppen von Salzkontaminationen in die zu isolierenden Nukleinsäuren und sind oftmals die Ursache dafür, dass eine Reihe von downstream-Applikationen (PCR, Restriktionsverdau, Hybridisierungen, Ligationen) nicht oder nur teilweise realisierbar sind. Darüber hinaus besteht beim Umgang mit chaotropen Puffern ein erhebliches gesundheitliches Risko (insbesondere bei Langzeitanwendungen) sowie eine erhebliche Umweltbelastung durch in Abwasser eingebrachte Schadstofflasten.

In der Patentschrift DE 198 56 064 wird zum erstenmal ein neuartiges Verfahren zur Isolierung von Nukleinsäuren beschrieben. Dabei erfolgt erstmals die Bindung der zu isolierenden Nukleinsäuren an mineralische Träger ohne die Verwendung der bisher dazu benötigten chaotropen Salze hoher Ionenstärken. Das Verfahren basiert (wie auch die chaotropen Verfahren) auf der Lyse des Ausgangsmaterials, der Bindung der Nukleinsäure an ein mineralisches Trägermaterial, dem nachfolgenden Waschen der gebundenen Nukleinsäuren mit ethanolhaltigen Waschpuffern, der Ethanolentfemung und der finalen Elution der Nukleinsäuren mit einem Elutionspuffer geringer Ionenstärke bzw. Wasser.
Für spezielle Protokolle z.B. der Isolierung von PCR-Fragmenten aus Amplifikationsansätzen wird der Lyseschritt nicht benötigt. Der PCR-Reaktionsansatz wird mit einem notwendigen Bindungspuffer versetzt und mit dem mineralischen Trägermaterial inkubiert. Anschließend erfolgt wieder ein Waschen mit einem ethanolhaltigen Waschpuffer, nachfolgend die Ethanolentfemung und final die Elution der gebundenen Nukleinsäure vom Trägermaterial.
Dass Verfahren ohne die Verwendung chaotroper Puffer deutliche Vorteile besitzen, zeigt sich daran, dass nach der Erstbeschreibung weitere Patentschriften auch die potenziellen Vorteile dieser neuen Bindungschemie beschreiben (z.B. DE 100 33 991).

Interessanterweise zeigt sich, dass alle weltweit kommerziell verfügbaren System zur Isolierung von Nukleinsäuren basierend auf der Bindung der Nukleinsäuren an mineralische Trägermaterialien (Magnetpartikel, Membranen, Carrier-Suspensionen u.a.) prinzipiell nach dem beschriebenen Verfahren arbeiten. Seit der Erstbeschreibung durch Vogelstein und Gillespie werden die gebundenen Nukleinsäuren immer mit Alkohol oder acetonhaltigen Salzlösungen gewaschen. Die Waschschritte sind essentieller Bestandteil der Extraktionsprotokolle und dienen neben der Entfernung von gebundenen unerwünschten inhibitorischen Stoffen immer auch der notwendigen Entfernung der für die Bindung der Nukleinsäuren notwendigen Salze.

Die Verwendung von Alkohol oder acetonhaltigem Waschpuffer bedeutet aber immer einen ganz erheblichen und bisher nicht gelösten Nachteil. Es ist bekannt, das selbst Spuren von Alkohol in der finalen Nukleinsäure downstream-Applikationen ganz erheblich beeinträchtigen kann. Deshalb ist es immer notwendig, einen Ethanolentfernungsschritt im eigentlichen Verfahren zur Isolierung oder Aufreinigung von Nukleinsäuren zu integrieren.
Dieser ist immer problematisch bei der Verwendung magnetischer Partikel oder partikulärer Carrier-Suspensionen, beansprucht einen erheblichen zeitlichen Aufwand und kann zu einem irreversiblen Verlust der Nukleinsäure führen, vor allem bei der Übertrocknung der Carrier-Materialien.
Besonders problematisch ist der Schritt der Entfernung von Restalkohol bei Applikationen zur Isolierung von Nukleinsäuren im Hochdurchsatzbereich.

Im Allgemeinen werden bis zu 30 min benötigt, um Membranen in Filterplatten oder magnetische Partikel im Rahmen von automatisierten Nukleinsäure-Reinigungsverfahren vom Restalkohol zu befreien.
Darüber hinaus sind auch die eigentlichen Waschschritte mit alkoholischen Waschpuffern gerade im Hochdurchsatzbereich zeitaufwendig und natürlich auch kostenintensiv.

Das Dokument EP 0580305 A2 beschreibt Verfahren und Erzeugnisse zur Aufreinigung von Nukleinsäuren. Ein zentraler Gesichtspunkt dieser Veröffentlichung besteht in der Beschreibung einer nichtproteinischen Polymermatrix aus Monomereinheiten, die aromatische Gruppen mit mindestens einer Hydroxylgruppe mit einem pKa kleiner als 9 umfassen (z.B. Poly-4-hydroxystyrol-beschichtetes Polyethylen - Beispiel 1). Die Autoren von EP 0580305 A2 beschreiben ferner Verfahren zur Aufreinigung von Nukleinsäure unter Verwendung der genannten Polymermatrix und stellen in diesem Zusammenhang detailliert den Einfluss von Kationen auf das Bindungsverhalten dieser besonderen Polymermatrix dar. Im Folgenden sind die Zusammenhänge kurz dargestellt:
(i): Die Polymermatrix aus EP 0580305 A2 bindet Proteine und Nukleinsäuren besser, je höher die Ionenstärke der Lösung ist (Spalte 7, Zeile 44-45 und Spalte 8, Zeile 1-3).
(ii) Mehrwertige Kationen haben stärkeren Einfluss auf die Bindungsaffinität als Einwertige (Spalte 5, Zeile 21-27 und Spalte 5, Zeile 37-38).
(iii) Mono- und Multivalente Kationen unterscheiden sich hinsichtlich ihres Einflusses auf die Bindungsaffinität nur quantitativ, sind qualitativ aber gleichwertig (Spalte 6, Zeile 5-8).

Das Dokument Lahiri DK et al. (Biochem Gen 31, 7/8, 321-328) beschreibt die Optimierung von Verfahren zur Isolierung und Lagerung von DNA aus humanen Blutproben. Zur DNA-Isolierung werden dabei zunächst die Zellen mit geeigneten Puffern lysiert. Anschließend wird die DNA durch Zusatz von Ethanol aus der Lösung gefällt (Seite 322, Materials and Methods). Eine Festphase kommt dabei nicht zum Einsatz.

Das Dokument WO 01/19980 A1 beschreibt Verfahren zum Anbinden von Nukleinsäuren an eine Festphase. Dabei kommen Festphasen zum Einsatz, die entweder sowohl hydrophobe als auch hydrophile Gruppen auf der Oberfläche tragen, oder Festphasen, die eine hydrophile Polymermatrix umfassen. Die Autoren von WO 01/19980 A1 machen Vorschläge für geeignete Waschlösungen, wobei als generische Waschlösung folgende Zusammensetzung vorgeschlagen wird: 50%-70% Ethanol oder 2-Propanol gegebenenfalls mit Zusätzen von EDTA, CDTA und TRIS in millimolaren Konzentrationen (Seite 12, Zeile 7-9). Ferner schlagen die Autoren nur noch eine weitere Waschlösung vor: Ammoniumacetat in 60%-80% Ethanol (Seite 12, Zeile 11-12). Die Autoren von WO 01/19980 A1 beschreiben ferner, dass eine reversible und sequenzunspezifische Bindung von Nukleinsäuren an die hydrophile Polymermatrix erreicht werden kann, wenn Wasser entziehende Reagenzien zugesetzt werden (Seite 16, Zeile 1-4). Besonders bevorzugt sollen dabei chaotrope Salze in hohen Konzentrationen zum Einsatz kommen (Seite 16, Zeile 18).

Aus diesen Nachteilen des bisherigen Standes leitet sich die Aufgabe ab, den Einsatz alkoholischer Komponenten zu vermeiden und damit eine erhebliche Verkürzung der Isolierungs- und Reinigungsverfahren zu erreichen.

Die Erfindung wird gemäß den Ansprüchen realisiert

Basierend auf der Verwendung nichtchaotroper Pufferformulierungen, wie in der Patentschrift DE 198 56 064 schon aufgeführt, zeigt sich, dass für die Bindung zu isolierender Nukleinsäuren nur unerwartet geringe Konzentrationen notwendig sind.

Der Kernpunkt der Erfindung liegt im gleichzeitigen Einsatz von mono- und von divalenten Kationen für die Bindung der Nukleinsäuren an die feste Phase.

Als monovalente Kation wird Na⁺, in Form der entsprechenden Salze eingesetzt. Als divalentes Kations wird Mg²⁺, in Form der entsprechenden Salze eingesetzt. Die Ausführungsform ist der kombinierte Einsatz vom Na⁺ und Mg²⁺.

Die mono- und divalenten Kationen können gemäß der Erfindung in den unterschiedlichsten Mengenverhältnissen eingesetzt werden. Der Erfolg tritt im breiten Bereich von mono-:divalenten Kationen im molaren Verhältnis von 6:4 bis 4:6, besonders bevorzugt ist die Ausführungsform mit gleichen (1:1) bzw. nahezu gleichen molaren Mengen von mono- und divalenten Kationen.

Die Gesamt-Kationenkonzentration in der Lösung vor der Bindung an die feste Phase ist < 0,5 M.

Wenn die Nukleinsäuren in einer Lösung vorliegen, die bereits mono- oder divalente Kationen enthält, z. B. nach einer vorangegangenen Lyse unterschiedlichster Ausgangsmaterialien, dann wird die vorhandene Menge der Kationen bei der Einstellung der gemäß der Erfindung optimalen Kationenkonzentration berücksichtigt. Wenn also der Lysepuffer divalente Kationen enthält, die nach der Lyse in der Lösung enthalten sind, wird nur noch die benötigte Menge monovalenter Kationen zugesetzt (und umgekehrt).

Überraschend ist dabei, dass bei der Kombination von Salzen eines monovalenten mit einem multivalenten Kation die für eine Bindung notwendigen Konzentrationen in einem Bereich liegen, in den Pufferformulierungen bestehend nur aus jeweils einem Salz nicht mehr für die Bindung ausreichend sind. So ermöglicht die Kombination von Magnesiumchlorid und Natriumchlorid noch bei Mengen von weniger als jeweils 5 mM jeweils die Bindung von Nukleinsäuren eines weiteren Größenspektrums (Beispiel 1). Die Verwendung der Mischung von Salzen monovalenter mit multivalenten Kationen als Bestandteile von Bindungspuffern für die Bindung von Nukleinsäuren an mineralische Träger ist bisher noch nicht beschrieben. Das überraschende Ergebnis ermöglicht nunmehr eine völlig neue Strategie zur Isolierung und Aufreinigung von Nukleinsäuren aus komplexen Ausgangsproben. Da überraschenderweise nur noch extrem geringe Salzkonzentrationen in Bindungspuffern notwendig sind, wird es möglich, Nukleinsäuren aus komplexen Proben oder aus Lösungen, welche eine Vielzahl an zu entfernenden Stoffen enthalten, gänzlich ohne einen Waschschritt zu isolieren.
Dies hat enorme Vorteile für die Isolierung von Nukleinsäuren und löst die geschilderten Probleme der Verwendung von alkoholhaltigen Waschpuffern insbesondere bei automatisierten Hochdurchsatzanwendungen in idealster Weise. So ermöglicht die Verwendung der erfindungsgemäßen Pufferformulierungen z.B. die Aufreinigung von PCR-Produkten aus einem komplexen PCR-Reaktionsansatz für eine nachfolgende empfindliche Sequenzierungsreaktion und ohne einen einzigen Waschschritt in Form einer automatisierten Applikation (Bindung der PCR-Produkte an die Filtermembran einer 96-Well-Platte) in weniger als 10 min. Bisherige Verfahren auf der Basis der Bindung der Nukleinsäure an eine feste Phase benötigen ca. 45 min -1h. Darüber hinaus ist der Verfahrensablauf nunmehr extrem einfach und beinhaltet lediglich das Mischen des PCR-Ansatzes mit einem der erfindungsgemäßen Bindungspuffer, das Überführen des Ansatzes auf die Filterplatte, das Durchsaugen der Lösung und die nachfolgend Elution der PCR-Produkte mittels Wasser bzw. mittels einer 10 mM Tris gepufferten wässrigen Lösung. Damit können PCR-Produkte extrem zeitsparend, ungefährlich und preiswert aufgereinigt werden. Der Durchsatz kann dabei dramatisch erhöht werden (auch bei sinkendem apparativen Aufwand; z.B. wird kein Wasch-Tool mehr bei einem Roboter benötigt). Die Qualität der aufgereinigten PCR-Produkte ist sehr hoch, was sich an den sauberen Sequenzreaktionen zeigt (Beispiel 2).

Weiterhin zeigt sich überraschender Weise, dass man Nukleinsäuren auch aus komplexen biologischen Proben in exzellenter Qualiät und Quantität auch ohne Waschschritte isolieren kann. Die in der Offenlegungsschrift DE 100 33 991 in einem Beispiel beschriebene Isolierung einer Plasmid DNA ohne einen Waschschritt erfolgte nicht aus dem zuvor hergestellten, geklärten Lysat. Die zu isolierende Plasmid-DNA wurde mittels bekannter Standardverfahren erst in reiner Form hergestellt und diese Plasmid-DNA nochmals mit einem Puffer inkubiert, an eine feste Phase gebunden und nachfolgend nach der notwendigen Entfernung des Alkohols des Bindungspuffers wieder von der festen Phase abgelöst.

Mit der vorliegenden Erfindung ist es nunmehr möglich, Plasmid-DNA direkt aus dem geklärten Lysat aufzureinigen wobei die Isolierung der Plasmid DNA auch ohne Waschschritt direkt nach der erfolgten Bindung an eine feste Phase erfolgen kann. Die Plasmid DNA ist dabei wiederum von exzellenter Qualität und Quantität (Vergleichsbeispiel 3).

Die Erfindung gestattet es weiterhin, auch extrem schnell, preiswert und einfach genomische Nukleinsäuren aus komplexen biologischen Proben zu isolieren. So wird lediglich ein Standardaufschluß des Ausgangsmaterials mittels z.B. eines klassischen Protease K-Verdaus in einem dafür kompatiblen Puffer durchgeführt, anschließend die lysierte Probe mit einem der erfindungsgemäßen nichtchaotropen Bindungspuffer versetzt und der Ansatz mit einer Nukleinsäure bindenden festen Phase inkubiert, ohne einen Waschschritt und nachfolgend die genomische Nukleinsäure mittels Wasser oder einer Tris-Lösung von der festen Phase isoliert. Die Qualität der isolierten DNA ist wiederum sehr hoch, sie ist sofort für weitere Applikationen einsetzbar.

Eine weitere Variante der vorliegenden Erfindung ergibt sich aus der Beobachtung, dass der pH-Wert der verwendeten Bindungspuffer von wesentlichen Einfluss auf sowohl die Ausbeute an den zu gewinnenden Nukleinsäuren ist als auch eine Selektivität gegenüber den Fragmentlängen von z.B. aufzureinigenden PCR-Produkten besitzt. Dabei ist es nicht notwendig mono- und multivalente Salze in einer Lösung mit einander zu kombinieren.

Dies ermöglicht es, über die Wahl des pH-Wertes des Bindungspuffers z.B. kleine PCR-Produkte von der Isolierung auszuschließen. Darüber hinaus zeigt sich auch, dass über die Kombination von Salz und Alkohol im Bindungspuffer im Zusammenhang mit der Wahl des pH-Wertes überraschende Effekte sichtbar werden. Die alkoholische Komponente im Bindungspuffer bewirkt eine Selektivität im Hinblick auf die Größenfraktionierung von DNA-Fragmenten. Tendenziell bewirkt eine Reduktion des pH-Wertes des Bindungspuffers eine Reduzierung der Ausbeute von kleineren DNA-Fragmenten bis hin zur kompletten Inhibition der Rückgewinnung. Dies ist besonders dann bedeutsam, wenn heterogene Probengemische vorliegen.

So kann bei pH-Werten >8 eine fast quantitative Rückgewinnung von DNA-Fragmenten eines breiten Größenspektrums (100 bp bis 10 000 bp) mit Bindungspuffern erreicht werden, wenn der Bindungspuffer eine alkoholische Komponente enthält.
Bei Bindungspuffern mit Alkohol wird der pH-Wert auf 5-9,5 und besonders bevorzugt auf 8-9,5, 6,5-8 bzw. 5-6,5 eingestellt. Damit ist die Erfassung bestimmter Fragmentgrößen möglich, d.h. bestimmte Fragmentgrößen werden nicht zurückgewonnen. Es sei hier darauf verwiesen, dass die Formulierung "keine Rückgewinnung" sich nicht als absolut versteht, d.h. Spuren an Nukleinsäurefragmente immer unspezifisch zurückgewonnen werden könnten.
Bei Bindungspuffern ohne Alkohol erfolgt eine Rückgewinnung von DNA-Fragmenten in quantitativen Mengen und über das Größenspektrum 100 bp bis 10 000 bp bevorzugt bei einem pH-Wert von >8,5. Letztlich ermöglicht die vorliegende Erfindung über die Kombination der Salzkomponenten und Alkoholkomponente sowie die Modifizierung des pH-Wertes, Bindungspuffer zu entwickeln, welche es erlauben, eine selektive Rückgewinnung von bestimmten Nukleinsäurefragmenten aus heterogenen Ausgangsproben zu ermöglichen.
Die vorliegende Erfindung ermöglicht somit in universeller Form eine deutliche Vereinfachung von Verfahren zur Isolierung von Nukleinsäuren aus Komplexen die nukleinsäureenthaltenden Proben.
Das Verfahren benötigt keine toxischen Chemikalien mehr, die eingesetzten Mengen an Salzen sind dramatisch reduziert, was zu einer deutlichen Umweltentlastung führt, die Verfahren benötigen weniger Verfahrensschritte und sind dadurch deutlich schneller als alle bisher verwendeten Techniken. Insbesondere im Hochdurchsatz stehen nunmehr preiswerte und extrem schnelle Verfahren zur Verfügung. Über die Zusammensetzung der Pufferformulierungen kann darüber hinaus eine selektive Größenfraktionierung von zurückzugewinnenden DNA-Fragmenten erreicht werden.

Die Erfindung wird nachfolgend anhand von Ausführungsbeispielen erklärt. Die Ausführungsbeispiele sollen aber keine Limitierung der Erfindung darstellen.

### Ausführungsbeispiele

### Beispiel 1:

Aufreinigung eines Spektrums an DNA-Fragmenten aus einer wässrigen Lösung Vergleich verschiedener Bindungspuffer hinsichtlich der Bindungseffizienz.

Puffer TH 1(5 mM NaCl; 5 mM MgCl₂/Tris HCl, Isopropanol)
Puffer TH 2 (10 mM MgCl₂/Tris HCl, Isopropanol)
Puffer TH 3 (10 mM NaCl/Tris HCl, Isopropanol)

Der Puffer TH1 ist eine Kombination aus einem monovalenten und einem divalenten Salz bei einer Gesamtionenstärke von 10 mM. Die Puffer TH2 und TH3 enthalten nur jeweils ein Salz (mit einem monovalenten Kation bzw. mit einem divalenten Kation) bei einer Gesamtionenstärke von 10 mM.
130 µl der jeweiligen Puffer wurden mit einer eine kommerzielle DNA-Leiter (Fa. FERMENTAS) enthaltenen wässrigen Lösung von 50 µl gemischt und der Ansatz auf eine Zentrifugationssäule mit einem Glasfaserfließ überführt. Anschließend wurde 1 min bei 10 000 rpm zentrifugiert und die Zentrifugationssäule in ein neues Reaktionsgefäß überführt. Die Elution der gebundenen Fragmente erfolgt durch die Zugabe von 30 µl einer 10 mM Tris-HCl-Lösung und nachfolgender Zentrifugation
für 1 min. Die Gesamtzeit der Isolierung der DNA-Fragmente betrug damit nur ca. 2 min. Die erhaltenen Eluate wurden auf ein 1,2 % Agarosegel aufgetragen und elektrophoretisch aufgetrennt. Wie in der elektrophoretischen Darstellung deutlich zu sehen ist, erfolgt eine effiziente Rückgewinnung der DNA-Fragmente nur mit dem Kombinationspuffer. Die jeweils nur ein Salz enthaltenen Bindungspuffer dagegen zeigen nur noch eine sehr geringe Bindungsvermittlung.

### (Abbildung 1)

### Vergleichsbeispiel 2:

Aufreinigung von PCR-Produkten aus einem komplexen PCR-Reaktionsansatz und nachfolgende Verwendung der aufgereinigten PCR-Produkte für eine DNA-Sequenzierung.

50 µl PCR-Reaktionsansätze wurden mit 130µl Bindungspuffer TH1 und TH4 (50 mM NaCl; 50 mM MgCl₂ /Tris HCl, Isopropanol) versetzt, nachfolgend auf eine Zentrifugationssäule mit Glasfaserfließ überführt, für 1 Minute zentrifugiert und abschließend die DNA wieder mittels 10 mM Tris HCl von der Säule eluiert. Die isolierten PCR-Produkte wurden dann für die Sequenzierung eingesetzt. Die Sequenzierungsergebnisse belegen, dass ohne die Verwendung von bisher notwendigen Waschschritten alle störenden Komponenten effizient entfernt wurden und eine hochreine DNA vorliegt.

### (Abbildungen 2-5)

### Vergleichsbeispiel 3:

Isolierung von Plasmid DNA aus bakteriellen Lysaten. Vergleich der Reinheit der isolierten Plasmid DNA bei unterschiedlichen Waschbedingungen bzw. ohne einen Waschschritt.

2 ml einer bakteriellen Übernachtkultur (XL-1 mit Plasmid pGEM) wurden zentrifugiert und das Pellet mit 200 µl Solution I (Tris, EDTA, Rnase A) resuspendiert. Danach erfolgte die Zugabe von 200 µl Solution II (SDS/NaOH). Die Reaktionsgefäße wurden mehrmals kurz vorsichtig geschüttelt. Nachfolgend erfolgte die Zugabe 200 µl einer Solution III (250 mM MgCl₂/Tris HCl). Die Reaktionsgefäße wurden kurz und vorsichtig geschüttelt und für 5 min bei Maximalgeschwindigkeit zenrifugiert. Der geklärte Überstand wurde mit 100 µl Isopropanol gemischt und auf eine Zentrifugationssäule mit einem Glasfaserfließ gegeben und für 1 min zentrifugiert. Nachfolgend wurden jeweils 3 Proben sofort mit 10 mM Tris versetzt (kein waschen), 3 Proben wurden mit 800 µl eines Waschpuffers ohne Alkohol (10mM NaCl/10mM MgCl₂/Tris HCl) versetzt und für 1 min zentrifugiert und nachfolgend die Plasmid-DNA mit 10 mM Tris-HCl von der Säule eluiert und 3 Proben wurden mit 70%igem Ethanol gewaschen, nachfolgend der Ethanol entfernt und die Plasmid DNA wiederum durch Zugabe von 10 mM Tris HCl eluiert.

Die nachfolgende Tabelle illustriert die notwendige Präparationszeit, die Qualität und Quantität der isolierte Plasmid DNA.

| **Probe** | **Waschschritt** | **Ratio 260:280** | **Ausbeute** | **Präparationszeit** |
|---|---|---|---|---|
| 1 | Kein Waschen | 1,71 | 12 µg | 8 min |
| 2 | Kein Waschen | 1,74 | 15 µg | 8 min |
| 3 | Kein Waschen | 1,81 | 14 µg | 8 min |
| 4 | Waschen ohne Alkohol | 1,82 | 15 µg | 10 min |
| 5 | Waschen ohne Alkohol | 1,81 | 14 µg | 10 min |
| 6 | Waschen ohne Alkohol | 1,84 | 12 µg | 10 min |
| 7 | Waschen mit Alkohol | 1,86 | 14 µg | 16 min |
| 8 | Waschen mit Alkohol | 1,92 | 16 µg | 16 min |
| 9 | Waschen mit Alkohol | 1,89 | 13 µg | 16 min |

### Vergleichsbeispiel 4:

Aufreinigung eines Spektrums an DNA-Fragmenten aus einer wässrigen Lösung.Vergleich verschiedener Bindungspuffer hinsichtlich der Bindungseffizienz in Abhängigkeit vom pH-Wert des Bindungspuffers.

50 µl einer wässrigen Lösung, enthaltend 2 µg DNA Ladder (Fermentas) wurden mit 130 µl
Bindungspuffer (R1-R8) ersetzt, nachfolgend auf eine Zentrifugationssäule mit Glasfaserfließ überführt, für 1 Minute zentrifugiert und abschließend die DNA wieder mittels 10 mM Tris HCl von der Säule eluiert. Die isolierten DNA-Fragmente wurden dann gelelektrophoretisch aufgetrennt.

### (Abbildung 6)

Bindungspuffer: enthaltend jeweils 50 mM MgCl₂ und optional 20% Isopropanol sowie 100 mM Tris HCl mit variierenden pH-Werten.

R1+ (pH 6,5/ mit Isopropanol)
R1 (pH 6,5/ ohne Isopropanol)
R2+ (pH 7,0/ mit Isopropanol)
R2 (pH 7,0/ ohne Isopropanol
R3+ (pH 7,5/ mit Isopropanol)
R3 (pH 7,5/ ohne Isopropanol
R4+ (pH 8,0/ mit Isopropanol)
R4 (pH 8,0/ ohne Isopropanol
R5+ (pH 8,5/ mit Isopropanol)
R5 (pH 8,5/ ohne Isopropanol
R6+ (pH 9,0/ mit Isopropanol)
R6 (pH 9,0/ ohne Isopropanol
R7+ (pH 9,5/ mit Isopropanol)
R7 (pH 9,5/ ohne Isopropanol
R8+ (pH 10,0/ mit Isopropanol)
R8 (pH 10,0/ ohne Isopropanol

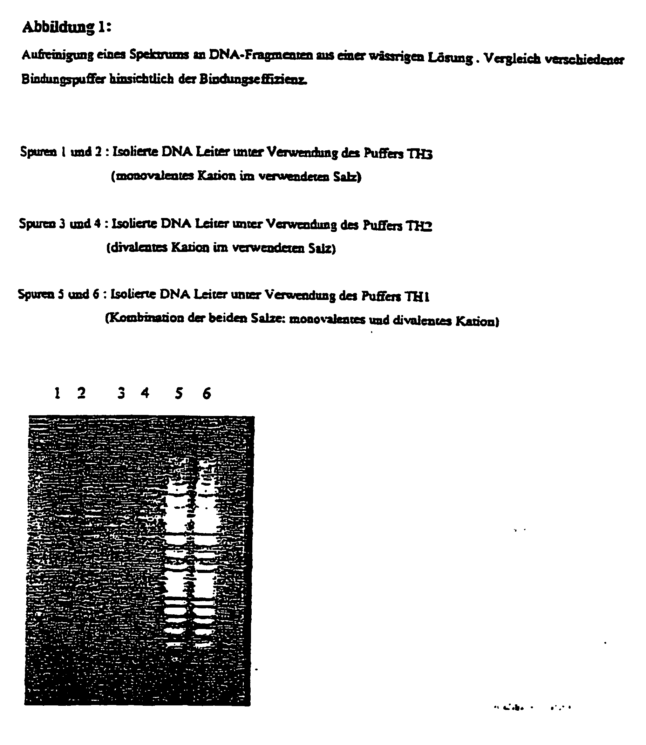

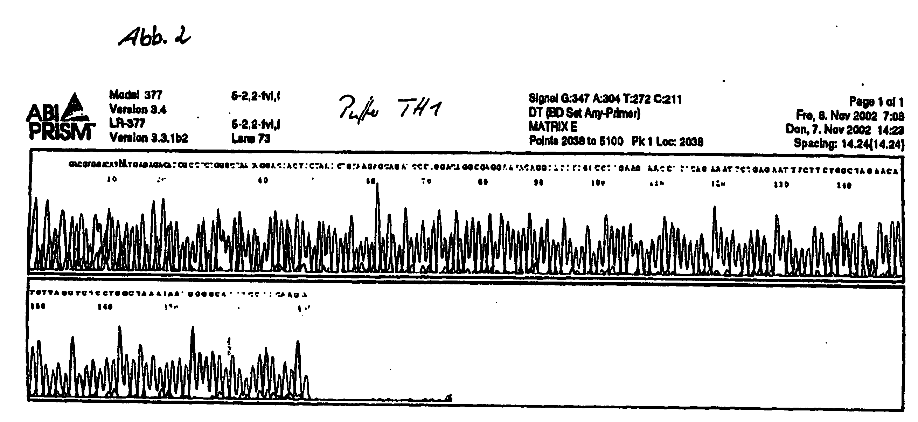

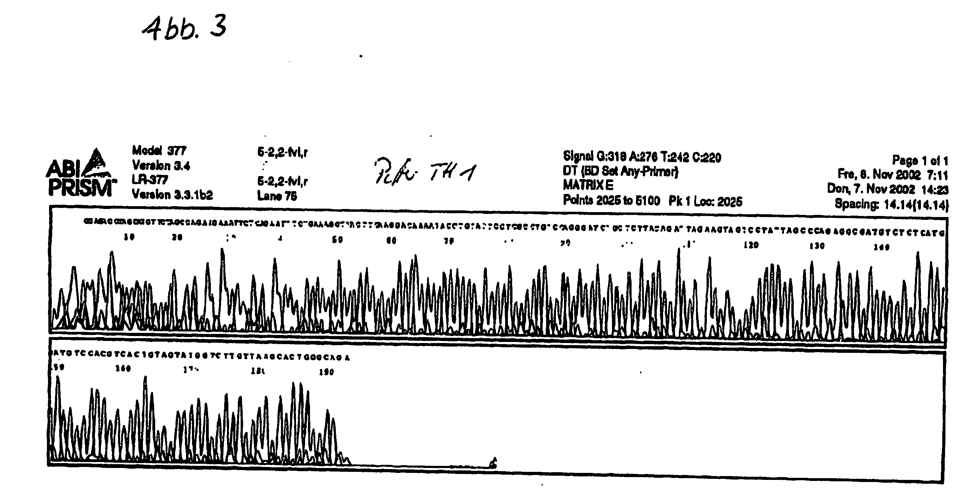

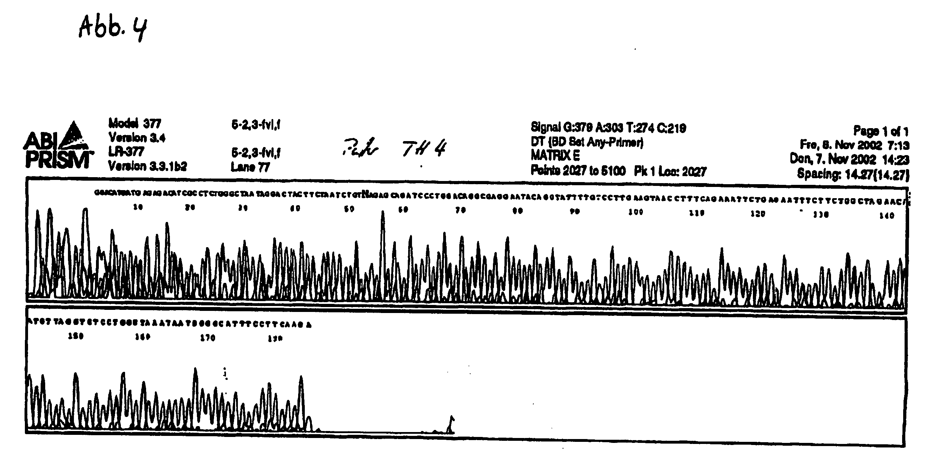

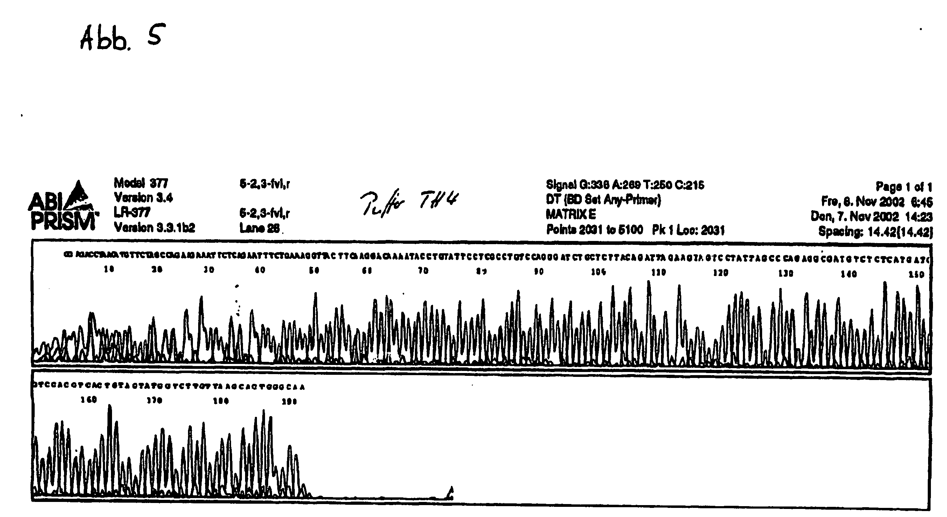

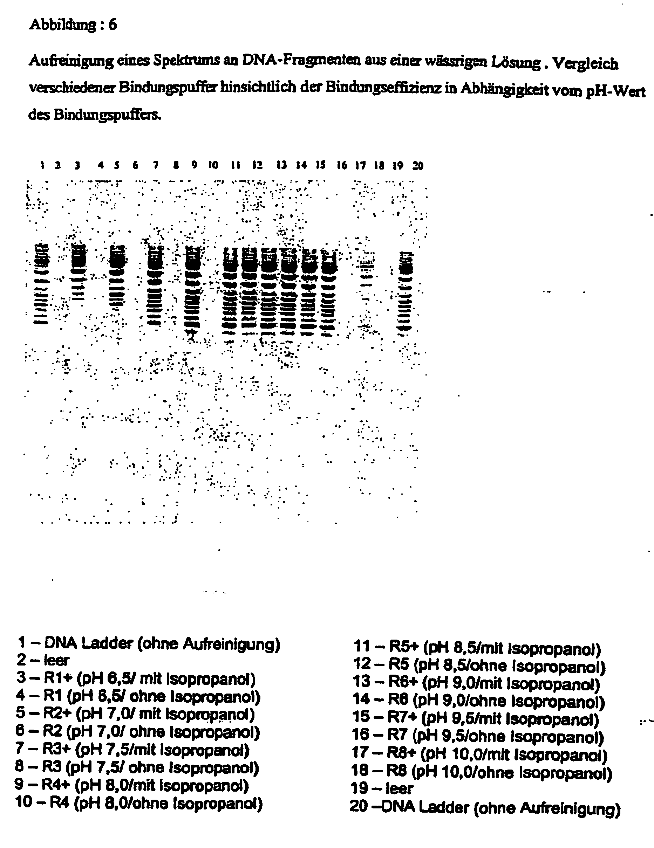

## Patentansprüche

1. Verfahren zur Isolierung von Nukleinsäuren aus einer Lösung durch Bindung an eine feste Phase, wobei man die Nukleinsäure enthaltende Lösung mit Zusätzen so einstellt, dass eine nicht-chaotrope Lösung enthaltend
- die zu isolierenden Nukleinsäuren
- Salze monovalenter und divalenter Kationen,
wobei als monovalente Salzkomponente Natriumchlorid in einer Konzentration von < 5 mM und als divalente Salzkomponente Magnesiumchlorid, in einer Konzentration von < 5 mM verwendet werden, und die Gesamtkonzentration dieser Salzkomponenten > 5 mM,
- Tris-HCl, und
- einen Alkohol
entsteht, welchen man danach mit der festen Phase in Kontakt bringt und ohne vorherigen Waschschritt die Nukleinsäure von der festen Phase eluiert,
wobei monovalente und divalente Salzkomponenten in der Lösung im molaren Mengenverhältnis 6:4 bis 4:6 und
bevorzugt im molaren Mengenverhältnis 1:1 bis nahezu 1:1 verwendet werden, und
wobei die feste Phase aus Bestandteilen besteht, die ausgewählt sind aus der folgenden Liste: SiO₂, Aerosile, magnetisierte Silikapartikel, gefällte Kieselsäure, pyrogene Kieselsäure, magnetische Silicapartikel, Glasfaservliese, Silicamembranen, oder Membranen, die funktionelle Gruppen tragen, die Glasfaservliesen oder Silicamembranen entsprechen.

2. Verfahren nach Anspruch 1, **dadurch gekennzeichnet, dass** der pH-Wert des Bindungspuffers mit Alkoholzusatz auf 6,5-9,5 eingestellt wird.

3. Verfahren nach einem der Ansprüche 1-2, **dadurch gekennzeichnet, dass** die Salzkomponenten Natriumchlorid und Magnesiumchlorid im molaren Verhältnis 1:1 verwendet werden.

4. Verfahren nach einem der Ansprüche 1-3, **dadurch gekennzeichnet, dass** als Alkohol Ethanol oder Isopropanol und/oder Polyethylenglykole unterschiedlicher Molekulargewichte, als weiterer Zusatz Polyvinylpyrrolidon eingesetzt werden.

5. Verfahren nach einem der Ansprüche 1-4, **dadurch gekennzeichnet, dass** als Elutionspuffer Wasser oder Wasser mit Tris-HCl-Zusatz verwendet werden.

6. Verfahren nach einem der Ansprüche 1-5, **dadurch gekennzeichnet, dass** der pH-Wert des Bindungspuffers mit Tris-HCl eingestellt wird.

## Claims

1. Method for the isolation of nucleic acids from a solution through binding to a solid phase, whereby the nucleic acid containing solution is adjusted with additives so that a non-chaotropic solution is formed, comprising
- the nucleic acids to be isolated,
- salts of monovalent and divalent cations,
whereby sodium chloride is used as the monovalent salt component at a concentration of < 5 mM and magnesium chloride is used as the divalent salt component at a concentration of < 5 mM, and the total concentration of these salt components is > 5 mM,
- Tris-HCl, and
- an alcohol,
which is subsequently brought into contact with the solid phase and the nucleic acids are eluted from the solid phase without a preceding wash step,
whereby monovalent and divalent salt components are used in the solution at a molar ratio of 6:4 to 4:6 and preferably at a molar ratio of 1:1 to almost 1:1, and
whereby the solid phase consists of components selected from the following list: SiO₂, aerosoles, magnetised silica particles, precipitated silicic acid, pyrogenic silicic acid, magnetic silica particles, glass fibre material, silica membranes or membranes, which carry functional groups that correspond to glass fibre material or silica membranes.

2. Method according to claim 1, **characterised in that** the pH value of the binding buffer with alcohol additive is adjusted to 6.5 - 9.5.

3. Method according to one of claims 1-2, **characterised in that** the salt components sodium chloride and magnesium chloride are used at a molar ratio of 1:1.

4. Method according to one of claims 1-3, **characterised in that** ethanol or isopropanol and/or polyethylene glycols of various molecular weights, or as an additional component polyvinylpyrrolidone is employed as alcohol.

5. Method according to one of claims 1-4, **characterised in that** water or water with Tris-HCl additive are used as elution buffer.

6. Method according to one of claims 1-5, **characterised in that** the pH value of the binding buffer is adjusted with Tris-HCl.

## Revendications

1. Procédé d'isolation d'acides nucléiques à partir d'une solution par liaison avec une phase solide, la solution contenant de l'acide nucléique étant ajustée avec des additifs de façon à obtenir une solution non-chaotropique contenant
- les acides nucléiques à isoler
- des sels de cations monovalents et divalents,
en tant que composants sel monovalents, du chlorure de sodium étant utilisé à une concentration de < 5mM et en tant que composants sel divalents, du chlorure de magnésium à une concentration de < 5 mM et la concentration globale de ces composants sel étant de > 5 mM,
- du Tris-HCl et
- un alcool
que l'on met ensuite en contact par une phase solide et que l'on élue l'acide nucléique de la phase solide sans étape de lavage préalable,
des composants sel monovalents et divalents étant utilisés dans un rapport molaire de 6:4 à 4:6 et de préférence dans un rapport molaire de 1:1 à presque 1:1, et
la phase solide se composant des éléments qui sont sélectionnés à partir de la liste suivante: SiO₂, aérosils, particules de silice magnétisées, acide silique précipitée, acide silique pyrogène, particules de silice magnétiques, nappes de fibres de verre, membranes silique ou membranes portant des groupes fonctionnels correspondant à des nappes de fibres de verre ou des membranes siliques.

2. Procédé selon la revendication 1 **caractérisé en ce que** la valeur pH du tampon de liaison est ajustée avec un additif d'alcool à 6,5 - 9,5.

3. Procédé selon l'une des revendications 1-2 **caractérisé en ce que** les composants sel chlorure de sodium et chlorure de magnésium sont utilisés à un rapport molaire de 1:1.

4. Procédé selon l'une des revendications 1-3 **caractérisé en ce que** comme alcool, on utilise de l'éthanol ou de l'isopropanol et/ou des polyéthylèneglycols de masses moléculaires différentes comme autre additif de la polyvinylpyrrolidone.

5. Procédé selon l'une des revendications 1-4 **caractérisé en ce que**, comme tampon d'élution, on utilise de l'eau ou de l'eau avec additif Tris-HCl.

6. Procédé selon l'une des revendications 1-5 **caractérisé en ce que** la valeur pH du tampon de liaison est ajustée avec un additif Tris-HCl.
